# EUROPEAN PATENT SPECIFICATION

(11) **EP 4 316 521 B1**
(45) Date of publication and mention of the grant of the patent: **18.06.2025**
(21) Application number: 23202349.9
(22) Date of filing: 25.03.2020
(51) Int. Cl.: A61K 47/68, A61K 47/65, A61K 9/19, A61P 35/00, A61K 9/08, A61K 38/00, A61K 39/395, A61K 47/20

(54) **ANTI-HER2 ANTIBODY DRUG CONJUGATE PHARMACEUTICAL PREPARATION**
PHARMAZEUTISCHES PRÄPARAT MIT ANTI-HER2-ANTIKÖRPER-WIRKSTOFFKONJUGAT
PRÉPARATION PHARMACEUTIQUE DE CONJUGUÉ ANTICORPS ANTI-HER2 MÉDICAMENT

(30) Priority: 26.03.2019 CN 201910231203
(43) Date of publication of application: 07.02.2024
(62) Divisional of application: 20776430.9
(73) Proprietor: RemeGen Co., Ltd., Shandong 264006 (CN)
(72) Inventor: LI, Zhuanglin, Shandong, 264006 (CN); XU, Qiaoyu, Shandong, 264006 (CN); YAO, Xuejing, Shandong, 264006 (CN); BAO, Qianjing, Shandong, 264006 (CN); FANG, Jianmin, Shandong, 264006 (CN)
(74) Representative: J A Kemp LLP

(56) References cited:
- WO-A1-2014/143765
- WO-A1-2017/009255
- WO-A1-2019/039483
- DAUGHERTY ANN L ET AL: "CHAPTER 8: Formulation and delivery issues for monoclonal antibody therapeutics", 1 January 2010, CURRENT TRENDS IN MONOCLONAL ANTIBODY DEVELOPMENT AND MANUFACTURING, SPRINGER, US, PAGE(S) 103 - 129, ISBN: 978-0-387-76642-3, XP009180430

## Description

### FIELD

The present invention relates to a pharmaceutical formulation of Her2 antibody-drug conjugate, which belongs to the field of antitumor drugs.

### BACKGROUND

Tumor-targeted biotherapy has attracted more and more attention in the research of cancer treatment. Among them, monoclonal antibody therapy has a high specificity against the target and low side effects, but has a relatively limited efficacy when used alone. Currently, the most successfully anti-tumor monoclonal antibody drugs are those targeting lymphocytic tumors, such as non-Hodgkin's lymphoma (NHL), chronic lymphocytic leukemia (CLL) and so on.

ErbB2, also known as Her2/neu, is the second member of the EGFR family, which plays a biological role by forming a heterodimer with the other three members of the EGFR family. The neu gene encoding ErbB2 was first isolated from rat neuroblastoma. The homologous gene of the neu gene in human somatic cells, called Her2, is located on the long arm of chromosome 17 (17q21.1). ErbB2, the product encoded by Her2, consists of 1255 amino acids and has a molecular weight of about 185 kDa, in which positions 720-987 is the tyrosine kinase active domain. In addition to acting through the PI3K and MAPK signaling pathways, ErbB2 can reduce the expression of cyclin D and c-myc, thereby reducing the expression of cyclin-dependent kinase (CDK) inhibitor p27kipl. The activity of CDK2 is inhibited, leading to cell proliferation. With the continuous and in-depth research, it has been found that HER2 is expressed and overexpressed in a variety of tumors. Therefore, there is an urgent need for drugs targeting HER2 to effectively treat malignant tumors. There are currently three monoclonal antibodies targeting Her2 on the market (see Table 1).

**Table 1 FDA approved monoclonal antibody drugs targeting Her2**

| Trade Name | Common Name | Company | Target | Indication | FDA Approved Date |
|---|---|---|---|---|---|
| Herceptin | Trastuzumab | Roche | Her-2 | Breast Cancer | 1998 |
| Perjeta | Pertuzumab | Genentech | Her-2 | Breast Cancer | 2012 |
| ogivri | trastuzumab-dkst | Mylan and Biocon company | Her-2 | Breast Cancer, Metastatic Gastric Cancer | 2017 |

Based on the characteristics of antibody targeting, a new generation of biological targeting drugs, antibody-drug conjugate (ADC), has emerged. ADC consists of three parts: antibody, cytotoxin, and a linker connecting the two together. After conjugating the monoclonal antibody to the cytotoxin, the antibody-drug conjugate utilizes the targeting of the monoclonal antibody to specifically recognize the receptor on the surface of the cancer cell, binds to the receptor, and then enters the cell, and releases a cytotoxic substance via the intracellular protease to stop cancer cells from reproducing and kill them. In the prior art, mammalian cell culture is generally used to produce an antibody, and the highly purified antibody is conjugated to the cytotoxin such as MMAE via a linker to obtain the antibody-drug conjugate (ADC). Antibody drug conjugating technology integrates a small molecule toxin drug and a biological protein, so that it owns the advantages of both, becoming a new generation of therapeutic product, which greatly enhances the efficacy of the drug while reducing toxic and side effects. Currently, there are four ADCs drugs on the market approved by the US FDA (see Table 2).

**Table 2 Four ADC drugs on the market**

| Trade Name | Common Name | Company | Target | Indication | FDA Approved Date |
|---|---|---|---|---|---|
| Mylotarg | Gemtuzumab Ozogamicin | Pfizer | CD33 | Acute Skeletal Leukemia | 2000/2017 |
| Adcetris | Brentuximab Vedotin | Seattle Genetics | CD30 | Hodgkin lymphoma, Anaplastic Large Cell Lymphoma | 2011 |
| Kadcyla | Ado-Trastuzumab Emtansine | Roche | Her-2 | Breast Cancer | 2013 |
| Besponsa | Inotuzumab Ozogamicin | Pfizer | CD22 | Acute Lymphoblastic Leukemia | 2017 |

Like other biomacromolecule drugs, ADCs are prone to degradation such as oxidation, deamidation and fragmentation, or forming microparticles and aggregates. In addition, conjugation itself may reduce the stability and change the physicochemical properties of the antibody. For example, the conjugating of DM1 to the anti-HER2 antibody trastuzumab results in the loss of stability of the CH2 domain of the antibody (Reference 1: Physicochemical Stability of the Antibody-Drug Conjugate Trastuzumab-DM1: Changes due to Modification and Conjugation Processes. Aditya A. Wakankar et al., Bioconjugate Chemistry 2010: 21 (9), 1588-1595). ADC drug is generally hydrophobic and as a whole may be more insoluble than an unconjugated antibody, and therefore become more prone to aggregation, forming microparticles or surface adsorption. In order to provide ADC drugs that are stable during transportation and storage, each adjuvant ingredient in the pharmaceutical formulation must be carefully selected. The adjuvant ingredients of ADCs currently on the market are as shown in Table 3. Since the types of adjuvant ingredient used in the ADC formulation currently on the market are very limited and there are many choices of the related ingredient of the formulation, the development of an adjuvant combination for the ADC formulation that are stable during transportation and storage involves the screening of a large number of adjuvant ingredients, also, the determination of the appropriate concentration requires a lot of effort and time.

**Table 3 Adjuvant ingredients of ADCs on the market**

| Trade Name | Common Name | Adjuvant Ingredient |
|---|---|---|
| Mylotarg | Gemtuzumab Ozogamicin | Dextran 40 |
| | | Sucrose |
| | | Sodium chloride |
| | | Monobasic sodium phosphate monohydrate |
| | | Dibasic sodium phosphate anhydrous |
| Adcetris | Brentuximab Vedotin | Citric acid monohydrate |
| | | Sodium citrate dihydrate |
| | | α, α-Trehalose dihydrate |
| | | Polysorbate 80 |
| Kadcyla | Ado-Trastuzumab Emtansine | Sucrose |
| | | Succinic Acid |
| | | Polysorbate20 |
| | | Sodium hydroxide |
| Besponsa | Inotuzumab Ozogamicin | Sucrose |
| | | Polysorbate 80 |
| | | Sodium chloride |
| | | Tromethamine |

It can also be seen from the adjuvant ingredients of the above four ADC formulations that each formulation is unique in the adjuvant ingredients used. Due to the low stability and complicated structure of monoclonal antibody drugs, it is extremely challenging to manufacture and store such drugs. Due to the heterogeneous structure of antibodies, in particular the complementarity determining regions (CDRs) and Fc glycosylation, the development of different monoclonal antibody formulations needs to be carried out individually based on case (Reference 2: Monoclonal antibodies: formulations of marketed products and recent advances in novel delivery system, Yanan Cui et al., Drug Development and Industrial Pharmacy, Volume 43, No. 4, Pages 519-530, 2017). In addition, the development of formulations for ADCs are more unique because the further involvement of conjugation and a toxin molecule.

The patent application (CN105008398A or WO2015074528A1) discloses a humanized RC48 antibody-drug conjugate, wherein the humanized RC48 antibody is a monoclonal antibody targeting Her2 (an antibody secreted from the hamster ovary cells (CHO cells) which were deposited at the China Center for Type Culture Collection with the deposit number of C2013170 or an antibody derived therefrom), and the cytotoxin is Monomethyl Auristatin E (MMAE), which is a derivative of dolastatin. The linker in this ADC is Maleimido-Caproyl-Valine-Citrulline-p-AminoBenzyloxy (mc-VC-PAB), and the recombinant humanized Her2 monoclonal antibody-MMAE conjugate is formed by the monoclonal antibody and MMAE linked to the linker through cysteine. The drug has achieved good therapeutic effects in Her2 positive tumor. Patent document WO2014143765 discloses a formulation comprising an anti-Epidermal Growth Factor Receptor (EGFR) antibody drug conjugate (ADC), a sugar, histidine, and a surfactant, wherein said formulation has a pH of about 5 - 7, and wherein said anti-EGFR ADC comprises an anti-EGFR antibody, or antigen-binding portion thereof, conjugated to an auristatin.

The preliminary experiments showed that, after purification, the recombinant humanized anti-Her2 monoclonal antibody-MMAE conjugate has a poor solubility in the conventional buffer, and visible insoluble microparticles will appear, which does not meet the standards for injection. However, if a clinically effective therapeutic dose is to be achieved, the protein concentration needs to be above 5 mg/ml. Therefore, there is a need to solve the problem of insoluble particles while ensuring a certain effective concentration. In addition, when the protein solution is lyophilized under vacuum, it is necessary to add a lyoprotectant to protect the protein from being destroyed during lyophilization, and to ensure that the lyophilized powder has a good appearance at the same time. In order to avoid the slow degradation of lyophilized powder of the protein during long-term storage, it is necessary to add an appropriate protein stabilizer. Lots of experiments are needed to develop and determine the appropriate protein stabilizer.

Therefore, the purpose of the present invention is to develop a combination of adjuvants for anti-Her2 monoclonal antibody-MMAE conjugate through a wide range screening and concentration range screening to achieve the following technical effects: the anti-Her2 monoclonal antibody-MMAE conjugate can dissolve well before and after lyophilization, and both the insoluble microparticles and the visible precipitates meet the standards for human injection; in addition, the conjugate can be stable for a long time during lyophilization and storage, not be easily polymerized or degraded after reconstitution, and still maintains good biological activity.

### SUMMARY

The present invention provides an aqueous liquid pharmaceutical formulation of an antibody-drug conjugate, wherein the formulation comprises the antibody-drug conjugate, a non-reducing sugar, an amino acid, and a solubilizer; wherein the non-reducing sugar is selected from mannitol, sucrose, and a combination thereof; the amino acid is selected from histidine, histidine hydrochloride, and a combination thereof; and the solubilizer is polysorbate 80; wherein the antibody-drug conjugate is an anti-HER2 monoclonal antibody and the drug is MMAE, and wherein the antibody of the antibody-drug conjugate comprises a heavy chain and a light chain, the heavy chain comprises CDRs 1-3 having amino acid sequences as shown in SEQ ID NO: 1, 2 and 3, respectively, and wherein the light chain comprises CDRs 1-3 having amino acid sequences as shown in SEQ ID NO: 4, 5 and 6, respectively.

In some embodiments, the concentration of the mannitol is 100-300 mmol/L, preferably 190-300 mmol/L, more preferably 200-260 mmol/L, most preferably 240-260 mmol/L. The concentration of the sucrose is 0-100 mmol/L, preferably 40-100 mmol/L, more preferably 60-100 mmol/L, most preferably 40-60 mmol/L.

In some embodiments, the histidine is histidine hydrochloride at a concentration of 0-100 mmol/L, preferably 5-50 mmol/L, more preferably 5-20 mmol/L, most preferably 10 mmol/L. The arginine is arginine hydrochloride at a concentration of 0-160 mmol/L, preferably 20-100 mmol/L, more preferably 30-90 mmol/L, most preferably about 35 mmol/L.

In some embodiments, the content of the glycerol is 0-1%, preferably 0.2-0.5% (w/v). The mass percentage of Tween 80 is 0-0.02% (w/v).

Further, the antibody in the antibody-drug conjugate is an anti-HER2 monoclonal antibody; and the drug to which the antibody is conjugated MMAE.

Further, the anti-HER2 antibody-drug conjugate is an anti-HER2 monoclonal antibody-vc-MMAE, wherein the anti-HER2 monoclonal antibody is connected to MMAE via a linker vc, and the structure formed by the connection of the linker and MMAE is:

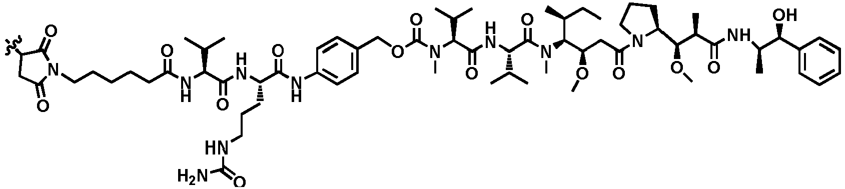

Further, the anti-HER2 monoclonal antibody comprises a heavy chain and a light chain,
(i) the heavy chain comprises CDRs 1-3 having amino acid sequences shown in SEQ ID NO: 1, 2 and 3, respectively; and
(ii) the light chain comprises CDRs 1-3 having amino acid sequences shown in SEQ ID NO: 4, 5 and 6, respectively.

Further, the monoclonal antibody is preferably a chimeric antibody or a humanized antibody.

In some embodiments, the concentration of the anti-HER2 monoclonal antibody-vc-MMAE is 5-30 mg/ml.

In some embodiments, the non-reducing sugar is 240-260 mmol/L of mannitol and/or 40 mmol/L-60 mmol/L of sucrose, the amino acid is 8-12 mmol/L of histidine hydrochloride, and the solubilizer is 0-0.02% (w/v) of Tween 80.

In some embodiments, wherein the non-reducing sugar is about 260 mmol/L of mannitol and about 40 mmol/L of sucrose, the amino acid is about 10 mmol/L of histidine hydrochloride, the concentration of the antibody-drug conjugate is about 10 mg/ml, and the solubilizer is about 0.02% (w/v) of Tween 80.

In some embodiments, wherein the non-reducing sugar is about 240 mmol/L of mannitol and about 60 mmol/L of sucrose, the amino acid is about 10 mmol/L of histidine hydrochloride, the concentration of the antibody-drug conjugate is about 10 mg/ml, and the solubilizer is about 0.02% (w/v) of Tween 80.

Further, the formulation has a pH of 4.5-7, preferably 5.6-6.8, more preferably 5.6-6.5, 5.6-6.4, 5.6-6.3, 6.1-6.4, or 6.1-6.3.

The pH of the formulation is adjusted with NaOH or hydrochloric acid.

In some embodiments, the lyophilized pharmaceutical formulation is obtained by lyophilization of the above aqueous liquid pharmaceutical formulation.

Further, before lyophilization, the aqueous liquid pharmaceutical formulation comprises about 260 mmol/L of mannitol, about 40 mmol/L of sucrose, about 10 mmol/L of histidine hydrochloride, about 0.02% (w/v) of Tween 80, and about 10 mg/ml of anti-HER2 monoclonal antibody-vc-MMAE, and has a pH of 5.6-6.8.

Further, before lyophilization, the aqueous liquid pharmaceutical formulation comprises about 240 mmol/L of mannitol, about 60 mmol/L of sucrose, about 10 mmol/L of histidine hydrochloride, 0.02% of Tween 80, and about 10 mg/ml of anti-HER2 monoclonal antibody-vc-MMAE, and has a pH of 5.6-6.8.

Further, before lyophilization, the non-reducing sugars comprised in the aqueous liquid pharmaceutical formulation are mannitol and sucrose at concentrations of about 47.36 mg/ml and about 13.69 mg/ml, respectively, and the amino acid is histidine hydrochloride at a concentration of about 2.10 mg/ml, and the solubilizer is Tween 80 at a content of about 0.02% (w/v).

Further, before lyophilization, the non-reducing sugars comprised in the aqueous liquid pharmaceutical formulation are mannitol and sucrose at concentrations of about 43.72 mg/ml and about 20.54 mg/ml, respectively, and the amino acid is histidine hydrochloride at a concentration of about 2.10 mg/ml, and the solubilizer is Tween 80 at a content of about 0.02% (w/v).

The present invention further provides the use of the above pharmaceutical formulations in the manufacture of a medicament for treating a disease caused by abnormal expression of Her2, preferably cancer; further preferably Her2 positive cancer; further preferably breast cancer, ovarian cancer, gastric cancer, urothelial cancer, gastroesophageal cancer, oesophageal cancer, endometrial cancer, lung cancer, or bladder cancer (Reference 3: Human Epidermal Growth Factor Receptor 2 (HER2) in Cancers: Overexpression and Therapeutic Implications, Nida Iqbal and Naveed Iqbal, Molecular Biology International, Volume 2014, Article ID 852748; and Reference 4: CN201810998055.4).

The present invention further provides a method for preparing a pharmaceutical formulation for an antibody-drug conjugate, comprising:
(1) preparing the formulation of any one of the above; and
(2) evaluating the stability of the antibody-drug conjugate in the formulation.

### BRIEF DESCRIPTION OF DRAWINGS

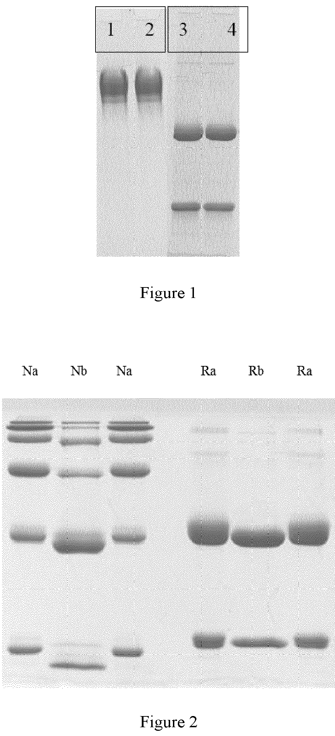

Figure 1 shows SDS-PAGE electrophoresis of the naked antibody after purification, in which, 1-2: non-reducing electrophoresis; and 3-4: reducing electrophoresis. The result in Figure 1 shows that the purity of the monoclonal antibody obtained in the experiment meets the requirements of further experiments.
Figure 2 shows 10% SDS-PAGE electrophoresis of ADC after conjugation, in which, Na: non-reducing electrophoresis; Nb: non-reducing electrophoresis after the antibody is reduced by TCEP; Ra: reducing electrophoresis of RC48-vc-MMAE; and Rb: reducing electrophoresis after the antibody is reduced by TCEP. The result of Figure 2 shows that the purity of the obtained ADC after conjugation meets the requirements of further experiments.

### EXAMPLES

### Example 1 Purification of Antibody

The murine-derived monoclonal antibody mRC48 and related humanized antibody RC48 were obtained based on the methods disclosed in the Example of the patent application (CN105008398A or WO2015074528A1), wherein RC48 comprised human IgG1κ heavy chain constant region and heavy chain variable region RC48-VH, and human IgG1κ light chain constant region and light chain variable region RC48-VL. Each of the above fragments was amplified and then subcloned into the expression vector pcDNA3.0, respectively. The constructed plasmids were transfected into suspended CHO cells (Invitrogen). The cells were cultured under standard conditions. When the nutrients in the medium were exhausted and the cells no longer grow, the culture mixture was collected. Then the cells were separated by centrifugation or filtration, and the supernatant containing the antibody protein collected and loaded onto the Protein A affinity chromatography column for the first purification. The eluted target protein was loaded onto a chromatographic column filled with cation packing for the second purification. The target protein was collected and then loaded onto the third column for the third purification in a target protein penetration mode. The purified protein that has passed the test on various indicators was then concentrated by ultrafiltration to obtain a protein with a concentration of about 20-30 mg/ml, which was the antibody protein stock solution and can be stored at -80°C for a long time.

Wherein, the CDR sequence of the RC48 antibody is as follows.

**Table 4 CDR sequences of RC48 antibody**

| | **Heavy Chain (VH)** | **Light Chain (VL)** |
|---|---|---|
| **CDR1** | **DYYIH (SEQ ID NO. 1)** | **KASQDVGTAVA (SEQ ID NO. 4)** |
| **CDR2** | **RVNPDHGDSYYNQKFKD (SEQ ID NO. 2)** | **WASIRHT (SEQ ID NO. 5)** |
| **CDR3** | **ARNYLFDHW (SEQ ID NO. 3)** | **HQFATYT (SEQ ID NO. 6)** |

### Example 2 Conjugation of Antibody to MMAE

### Conjugation of humanized antibody RC48 to drug molecules

Firstly, the stock solutions of TCEP (Tris-2-carboxyethyl-phosphine) and DTPA (Diethylene triamine pentacetate acid) were dissolved/diluted in the conjugation buffer respectively, and then mixed with the monoclonal antibody in a volume ratio of 1:1 (v:v=1:1), in which the molar ratio of the final concentration of TCEP to the antibody was 1.9:1, the final concentration of DTPA was 1 mmol/L, and the reaction was performed with stirring at 25 °C for 2 h. The reductive reaction with TCEP had a good reproducibility, and the numbers of free thiol could reach 3.5-4.5 after reduction.

After reduction with TCEP, the antibody may be directly subjected to subsequent conjugation. Drugs (vc-MMAE, vc-MMAF, mc-MMAF) were dissolved in DMSO (dimethyl sulfoxide) to a concentration of 10 mmol/L. The drugs were added slowly in the molar ratio of the drug to thiol of 1.1:1 to the antibody, and the reaction was performed with stirring at 25 °C for 2 h. The free thiol concentration was detected at 412 nm by DTNB method (close to zero), the residual unreacted drugs and free small molecules such as DMSO were removed by purification, and the conjugation result was determined by SDS-PAGE electrophoresis, SEC and HPLC methods. The conjugation reaction had a good reproducibility, and the open free thiol can be conjugated completely with a conjugation degree of 3.5-4.5.

### Example 3 Effect of pH on Dissolution and Purity of Recombinant humanized Her-2 Antibody-MMAE Conjugate (RC48-vc-MMAE)

### Experimental procedure

2 g of recombinant humanized anti-Her2 monoclonal antibody-MMAE conjugate for injection was ultrafiltered and dialyzed into a buffer containing 0.1 mol/L citric acid, 0.02 mol/L trihydroxymethyl aminomethane, 0.02 mol/L sodium dihydrogen phosphate and 0.15 mol/L sodium chloride in a dialysis ratio of not less than 10⁴ times. The obtained solution was divided into 13 parts equally, and each part was concentrated to a protein concentration of 10 mg/ml by a centrifugal ultrafiltration tube with a pore size of 30 kDa, and then the pH was adjusted to 4.2, 4.6, 5.0, 5.4, 5.6, 5.8, 6.0, 6.2, 6.4, 6.6, 6.8, 7.0, or 7.4 with hydrochloric acid or sodium hydroxide solution. The clarity was observed and the change in the concentration was detected (the results are as shown in Table 5). The samples were respectively filtered into sterile 20 ml penicillin bottles with a sterile syringe filter with a pore size of the membrane of 0.22 micron in the super clean bench, with 3 bottles of each pH value. Each bottle was sealed with a sterile rubber stopper and tied with an aluminum cap, and stored at 25 °C. Samples were taken with a sterile syringe at 0 hour, 1 day, 3 days, and 7 days for SEC-HPLC analysis and the results are as shown in Table 6.

Studies have found that the pH of the protein under experimental conditions has a great impact on the dissolution of the protein. The protein precipitated below pH 5.4 and dissolved well above pH 5.6; and the protein aggregates did not increase after 3 days and slightly increased after 7 days when stored below pH 6.8, increased with time above pH 7.0, and the higher the pH, the more the aggregates (see Table 5 and Table 6).

**Table 5 Clarity and protein concentration**

| pH | 0 hour | | 1 day | | 3 days | | 7 days | |
|---|---|---|---|---|---|---|---|---|
| | Clarity | Concentrati on (mg/ml) | Clarity | Concentrati on (mg/ml) | Clarity | Concentrati on (mg/ml) | Clarity | Concentration (mg/ml) |
| 4.2 | Massive Precipitates | 6.34 | Massive Precipita tes | 5.67 | Massive Precipit ates | 5.21 | Massive Precipit ates | 4.40 |
| 4.6 | Massive Precipitates | 6.63 | Massive Precipita tes | 5.53 | Massive Precipit ates | 5.46 | Massive Precipit ates | 4.82 |
| 5.0 | Slight Precipitates | 8.82 | Slight Precipita tes | 8.17 | Slight Precipit ates | 7.98 | Slight Precipit ates | 7.64 |
| 5.4 | Slight Precipitates | 8.99 | Slight Precipita tes | 8.78 | Slight Precipit ates | 8.45 | Slight Precipit ates | 8.10 |
| 5.6 | Clear | 9.99 | Clear | 10.01 | Clear | 10.00 | Clear | 9.96 |
| 5.8 | Clear | 10.03 | Clear | 10.00 | Clear | 9.98 | Clear | 9.97 |
| 6.0 | Clear | 9.97 | Clear | 9.99 | Clear | 10.01 | Clear | 10.00 |
| 6.2 | Clear | 10.00 | Clear | 9.98 | Clear | 9.97 | Clear | 9.98 |
| 6.4 | Clear | 9.95 | Clear | 9.97 | Clear | 9.98 | Clear | 10.00 |
| 6.6 | Clear | 9.97 | Clear | 9.96 | Clear | 10.02 | Clear | 9.95 |
| 6.8 | Clear | 10.05 | Clear | 10.01 | Clear | 9.96 | Clear | 9.97 |
| 7.0 | Clear | 10.02 | Clear | 10.01 | Clear | 10.00 | Clear | 10.03 |
| 7.4 | Clear | 10.00 | Clear | 10.03 | Clear | 9.99 | Clear | 9.97 |

**Table 6 Purity**

| pH | 0 hour (%) | | | 1 day (%) | | | 3 days (%) | | | 7 days (%) | | |
|---|---|---|---|---|---|---|---|---|---|---|---|---|
| | Purity | Aggregate | Degradation | Purity | Aggregate | Degradation | Purity | Aggregate | Degradation | Purity | Aggregate | Degradation |
| 5.6 | 99.6 | 0.4 | 0 | 99.5 | 0.5 | 0 | 99.2 | 0.8 | 0 | 98.5 | 1.5 | 0 |
| 5.8 | 99.5 | 0.5 | 0 | 99.7 | 0.3 | 0 | 99.1 | 0.9 | 0 | 98.3 | 1.7 | 0 |
| 6.0 | 99.4 | 0.6 | 0 | 99.5 | 0.5 | 0 | 99.1 | 0.9 | 0 | 98.4 | 1.6 | 0 |
| 6.2 | 99.6 | 0.4 | 0 | 99.3 | 0.7 | 0 | 99.0 | 1.0 | 0 | 98.5 | 1.5 | 0 |
| 6.4 | 99.5 | 0.5 | 0 | 99.3 | 0.7 | 0 | 99.2 | 0.8 | 0 | 98.3 | 1.7 | 0 |
| 6.6 | 99.5 | 0.5 | 0 | 99.4 | 0.6 | 0 | 99.2 | 0.8 | 0 | 98.3 | 1.7 | 0 |
| 6.8 | 99.6 | 0.4 | 0 | 99.5 | 0.5 | 0 | 99.0 | 1.0 | 0 | 98.2 | 1.8 | 0 |
| 7.0 | 99.5 | 0.5 | 0 | 99.1 | 0.9 | 0 | 98.6 | 1.4 | 0 | 97.1 | 2.9 | 0 |
| 7.4 | 99.6 | 0.4 | 0 | 98.3 | 1.7 | 0 | 97.2 | 2.8 | 0 | 96.4 | 3.6 | 0 |

### Example 4 Screening of Adjuvants

Through the analysis of a large amount of information and the screening through experiments in the early stage, sucrose, mannitol, glycerol, histidine, arginine, polysorbate 80 (i.e. Tween 80) and the like were preliminarily identified as candidate adjuvants for further screening, and the following tests were further carried out.

**Table 7 Screening of adjuvants for RC48-vc-MMAE injection (Formula Numbers A1, A3, A5, A7 are not according to the claims)**

| Formula Number | Histidine Hydrochloride (mmol/Lol/L) | Arginine (mmol/Lol/L) | Sucrose (mmol/Lol/L) | Mannitol (mmol/Lol/L) | Glycerol (%) | Tween 80 (%) | Protein (mg/ml) | pH |
|---|---|---|---|---|---|---|---|---|
| A1 | 10 | 35 | 0 | 240 | 0 | 0 | 10 | 6.3 |
| A2 | 10 | 35 | 0 | 240 | 0 | 0.02 | 10 | 6.3 |
| A3 | 10 | 0 | 60 | 240 | 0 | 0 | 10 | 6.3 |
| A4 | 10 | 0 | 60 | 240 | 0 | 0.02 | 10 | 6.3 |
| A5 | 10 | 0 | 0 | 200 | 1.0 | 0 | 10 | 6.3 |
| A 6 | 10 | 0 | 0 | 200 | 1.0 | 0.02 | 10 | 6.3 |
| A 7 | 10 | 0 | 0 | 300 | 0 | 0 | 10 | 6.3 |
| A 8 | 10 | 0 | 0 | 300 | 0 | 0.02 | 10 | 6.3 |

**Table 8 Clarity of RC48-vc-MMAE injection**

| Formula Number | 0 hour | 1 day | 3 days | 7 days |
|---|---|---|---|---|
| A1 | ++ | ++ | ++ | ++ |
| A 2 | - | - | - | - |
| A 3 | ++ | ++ | ++ | ++ |
| A 4 | - | - | - | - |
| A 5 | - | - | - | - |
| A 6 | - | - | - | - |
| A 7 | ++ | ++ | ++ | ++ |
| A 8 | - | - | - | - |

| | | | | |
|---|---|---|---|---|
| Note: "-" indicates that the number of white dots (visible precipitates) is less than 3, "+" indicates that the number of white dots (visible precipitates) is between 3-5, and "++" indicates that the number of white dots (visible precipitates) is more than 5. | | | | |

**Table 9 Purity of RC48-vc-MMAE injection**

| Formula Number | 0 hour(%) | 1 day (%) | 3 days (%) | 7 days (%) |
|---|---|---|---|---|
| A1 | 99.5 | 99.2 | 99.0 | 98.2 |
| A 2 | 99.6 | 99.1 | 98.8 | 98.3 |
| A 3 | 99.5 | 99.6 | 99.5 | 99.5 |
| A 4 | 99.7 | 99.6 | 99.6 | 99.6 |
| A 5 | 99.4 | 99.1 | 98.7 | 98.2 |
| A 6 | 99.5 | 98.9 | 98.6 | 98.3 |
| A 7 | 99.6 | 99.0 | 98.6 | 98.0 |
| A 8 | 99.5 | 98.9 | 98.3 | 98.2 |

It can be seen from the above results that the addition of arginine had no obvious effect on eliminating the visible precipitates of the protein. While the addition of sucrose, glycerol and polysorbate 80 can all improve the condition of the protein. Therefore, it was determined that an appropriate amount of sucrose may be added to the formulation to protect the protein, and glycerol and polysorbate 80 may be added to promote the dissolution of the protein.

### Example 5 Vacuum freeze drying

The protein stock solution was taken out from the -80°C refrigerator, thawed, diluted accurately with "1× formulation buffer" to a protein concentration of 10 mg/ml, and subpackaged into a sterile, pyrogen-free standard 20 ml lyophilized penicillin bottle, 6 ml per bottle, and then subjected to freeze drying in vacuum.

### Freeze drying conditions

Pre-freezing: -45 °C for 5 hours;
Primary drying: -26 °C for 40 hours, at vacuum degree of 10-15 Pa; and
Secondary drying: 25 °C for 10 hours, at vacuum degree of 10-15 Pa.

After lyophilization, the bottle was sealed with a rubber stopper in a vacuum state, taken out of the lyophilizer and put on an aluminum cap.

### Example 6: Screening Formulas

Based on the formulas designed in Table 10, the appearance and shaping stability of the lyophilized powders of the formulations were examined. After lyophilization, the samples were stored at 4 °C and 37 °C. The appearance of the samples was observed on days 0, 1, 3, and 7, respectively, and the corresponding formulas with better appearance and shape were screened out. The results are shown in Table 11.

**Table 10 List of buffer formulas for RC48-vc-MMAE injection (Formula Numbers B1, B3, B5, B7, B9, B11 are not according to the claims)**

| Formula Number | Histidine Hydrochloride (mmol/Lol/L) | Sucrose (mmol/Lol/L) | Mannitol (mmol/Lol/L) | Glycerol (%) | Tween 80 (%) | Protein (mg/ml) | pH |
|---|---|---|---|---|---|---|---|
| B1 | 10 | 40 | 260 | 0 | 0 | 10 | 6.3 |
| B 2 | 10 | 40 | 260 | 0 | 0.02 | 10 | 6.3 |
| B 3 | 10 | 60 | 240 | 0 | 0 | 10 | 6.3 |
| B 4 | 10 | 60 | 240 | 0 | 0.02 | 10 | 6.3 |
| B 5 | 10 | 100 | 200 | 0 | 0 | 10 | 6.3 |
| B 6 | 10 | 100 | 200 | 0 | 0.02 | 10 | 6.3 |
| B 7 | 10 | 40 | 240 | 0.2 | 0 | 10 | 6.3 |
| B 8 | 10 | 40 | 240 | 0.2 | 0.02 | 10 | 6.3 |
| B 9 | 10 | 60 | 190 | 0.5 | 0 | 10 | 6.3 |
| B 10 | 10 | 60 | 190 | 0.5 | 0.02 | 10 | 6.3 |
| B 11 | 10 | 100 | 100 | 1.0 | 0 | 10 | 6.3 |
| B 12 | 10 | 100 | 100 | 1.0 | 0.02 | 10 | 6.3 |

**Table 11 Appearance observation of lyophilized powders**

| Formula Number | Appearance | | | | | | | |
|---|---|---|---|---|---|---|---|---|
| | 4 °C | | | | 37 °C | | | |
| | 0 hour | 1 day | 3 days | 7 days | 0 hour | 1 day | 3 days | 7 days |
| B1 | ++ | ++ | ++ | ++ | ++ | ++ | ++ | ++ |
| B 2 | ++ | ++ | ++ | ++ | ++ | ++ | ++ | ++ |
| B 3 | ++ | ++ | ++ | ++ | ++ | ++ | ++ | ++ |
| B 4 | ++ | ++ | ++ | ++ | ++ | ++ | ++ | ++ |
| B 5 | ++ | ++ | ++ | ++ | ++ | ++ | ++ | ++ |
| B 6 | ++ | ++ | ++ | ++ | ++ | ++ | ++ | ++ |
| B 7 | ++ | ++ | ++ | ++ | ++ | + | + | - |
| B 8 | ++ | ++ | ++ | ++ | ++ | + | + | - |
| B 9 | ++ | ++ | ++ | ++ | ++ | + | + | - |
| B 10 | ++ | ++ | ++ | ++ | ++ | + | + | - |
| B11 | ++ | ++ | ++ | ++ | ++ | + | + | - |
| B 12 | ++ | ++ | ++ | ++ | ++ | + | + | - |

| | | | | | | | | |
|---|---|---|---|---|---|---|---|---|
| Note: 1) "- -" indicates that the volume of product is reduced to less than half of its volume before lyophilization; "-" indicates that the volume of product is reduced to more than half of its volume before lyophilization; "+" indicates that only the edge has slight shrinkage, and the volume of product is basically the same as that of before lyophilization; and "++" indicates that there is no volume change at all, and the volume of the product is the same as before lyophilization. 2) Qualified standards for the appearance of lyophilized powders are: uniform color, even and dense pores, and the volume and shape before and after lyophilization remain basically unchanged, showing a block or sponge-like structure. It can be seen from Table 11 that the formulation containing glycerol in the formula showed slight shrinkage after being placed at 37 °C for one day, and showed obvious shrinkage and collapse after being placed at 37 °C for seven days. Therefore, glycerol was excluded as an adjuvant for the ADC formulation. | | | | | | | | |

### Example 7 Determination of Formula and Stability Test of Lyophilized Powders

The formulas B7-B12 were eliminated after the appearance inspection for lyophilized powders. The adjuvants of formulas B1-B6 were further screened through the inspection for moisture content, visible precipitates, insoluble microparticles, and the stability.

The lyophilized powders of each formula were stored at 37 °C, 25 °C and 4 °C, and then sampling at different times and subjected to assays of SDS-PAGE, reverse phase HPLC, ligand binding assay, biological activity, moisture content, appearance, pH value, visible precipitates, insoluble microparticles and the like.

**Table 12 Appearance observation of lyophilized powders**

| Formula Number | B1 | B2 | B3 | B4 | B5 | B6 |
|---|---|---|---|---|---|---|
| Appearance | ++ | ++ | ++ | ++ | ++ | ++ |

| | | | | | | |
|---|---|---|---|---|---|---|
| Note: "- -" indicates that the volume of product is reduced to less than half of its volume before lyophilization; "-" indicates that the volume of product is reduced to more than half of its volume before lyophilization; "+" indicates that only the edge has slight shrinkage, and the volume is basically the same as before lyophilization; and "++" indicates that there is no shrinkage at all, and the volume of the product is the same as before lyophilization. | | | | | | |

It can be seen from Table 12 that the appearance of the lyophilized powders of formulas B1-B6 all meet the standard requirements.

Three samples were picked from each formula and tested for the moisture content according to the prescribed method. The average value of the moisture content was calculated and the results are shown in Table 12.

**Table 12 Moisture contents of lyophilized powders**

| Formula Number | B1 | B2 | B3 | B4 | B5 | B6 |
|---|---|---|---|---|---|---|
| Moisture Content (%) | 0.658 | 0.586 | 0.557 | 0.606 | 0.571 | 0.560 |

The moisture contents of formulas B1, B2, B3, B4, B5, and B6 were all less than 3%, which was qualified.

Five samples per formula were randomly chosen, and the visible precipitates were inspected after the sample was redissolved according to the prescribed method. Then, three of the five samples per formula were sampled and the insoluble microparticles were measured according to the prescribed method. The results are shown in Table 13.

**Table 13 Visible precipitates and insoluble microparticles after redissolution of lyophilized powders**

| Formula Number | Visible precipitate (Average Number) | Number of Insoluble Microparticle (>10 µm) |
|---|---|---|
| B1 | 4.3 | 7542 |
| B 2 | 0.6 | 642 |
| B 3 | 5.0 | 6792 |
| B 4 | 0.5 | 650 |
| B 5 | 7.7 | 9168 |
| B 6 | 1.2 | 926 |

It can be seen from the results of visible precipitates and insoluble microparticle that the formulas B1, B3, and B5 did not meet the requirements and were therefore excluded.

**Table 14 Results of stability after storage at 37 °C for 1 month**

| Formula Number | SEC-HP LC (%) | HIC-HPLC Average Conjugation Rate | Binding Activity (IC50)(pM) | Cell Viability (IC50)(pM) | Moisture Content (%) | Appeara nce | Number of Visible precipitate | pH | Insoluble Microparticle (>10 µm) | Insoluble Microparticle (>25 µm) |
|---|---|---|---|---|---|---|---|---|---|---|
| B2 | 99.02 | 3.98 | 15.23 | 65.2 | 0.889 | ++ | 1 | 6.30 | 669 | 9.0 |
| B4 | 99.18 | 3.99 | 12.59 | 57.6 | 0.896 | ++ | 0 | 6.30 | 645 | 6.0 |
| B6 | 99.12 | 3.96 | 14.77 | 57.8 | 0.902 | ++ | 6 | 6.30 | 6418 | 692.0 |

**Table 15 Results of stability after storage at 25 °C for 3 months**

| Formula Number | SEC-HP LC (%) | HIC-HPLC Average Conjugation Rate | Binding Activity (IC50)(pM) | Cell Viability (IC50)(pM) | Moisture Content (%) | Appea rance | Number of Visible precipitate s | pH | Insoluble Microparticle (>10 µm) | Insoluble Microparticle (>25 µm) |
|---|---|---|---|---|---|---|---|---|---|---|
| B2 | 99.12 | 4.03 | 13.27 | 55.2 | 0.789 | ++ | 0 | 6.30 | 300 | 12.0 |
| B4 | 99.25 | 4.05 | 12.68 | 56.1 | 0.796 | ++ | 0 | 6.30 | 315 | 6.0 |
| B6 | 99.34 | 4.05 | 12.91 | 55.6 | 0.806 | ++ | 6 | 6.30 | 6209 | 646.0 |

**Table 16 Results of stability after storage at 4 °C for 6 months**

| Formula Number | SEC-HP LC (%) | HIC-HPLC Average Conjugating Rate | Binding Activity (IC50)(pM) | Cell Viability (IC50)(pM) | Moisture Content (%) | Appea rance | Number of Visible precipitate | pH | Insoluble Microparticle (>10 µm) | Insoluble Microparticle (>25 µm) |
|---|---|---|---|---|---|---|---|---|---|---|
| B2 | 99.45 | 4.01 | 13.23 | 55.4 | 0.560 | ++ | 0 | 6.30 | 618 | 6.0 |
| B4 | 99.48 | 3.99 | 12.19 | 57.1 | 0.579 | ++ | 0 | 6.30 | 672 | 0.0 |
| B6 | 99.21 | 3.95 | 13.30 | 56.5 | 0.589 | ++ | 4 | 6.30 | 6120 | 610.0 |

| | | | | | | | | | | |
|---|---|---|---|---|---|---|---|---|---|---|
| Note: appearance standard: "- -" indicates that the volume of product is reduced to less than half of its volume before lyophilization; "-" indicates that the volume of product is reduced to more than half of its volume before lyophilization; "+" indicates that only the edge has slight shrinkage, and the volume of the product is basically the same as before lyophilization; and "++" indicates that there is no shrinkage at all, and the volume of the product is the same as before lyophilization. | | | | | | | | | | |

It can be seen from the above results that products of formulas B2 and B4 have better stability at different temperatures (4 °C, 25 °C, 37 °C), while product of formula B6 has many insoluble particles and was therefore excluded. It can be seen from the above tests that lyophilized powders of formulas B2 and B4 have an excellent performance in terms of appearance, moisture content, visible precipitates and insoluble microparticles after redissolution of lyophilized powder, and stability.

Based on the above experimental evidence, it can be seen that the selection of specific adjuvants in a class of substances such as non-reducing sugars, amino acids, and solubilizer has an unpredictable effect on the final formulation. For the Her2 monoclonal antibody-drug conjugate of the present invention, a large number of experiments are needed to test various properties in order to finally obtain a good combination of ingredients. For example, the addition of arginine has no obvious effect on eliminating the visible precipitates of the protein, and the addition of sucrose, glycerol and polysorbate 80 can all reduced the aggregation of the protein. However, the addition of glycerol will make the lyophilized product prone to show obvious shrinkage and collapse in the appearance of the reconstituted aqueous solution after reconstitution. In addition, too high concentration of sucrose or too low concentration of mannose will lead to an increase in insoluble matter after reconstruction. These results were difficult for those skilled in the art to predict before conducting relevant tests. In addition, since ADC formulation involves the combined use of multiple adjuvants, long-term stability tests and other factors, this makes it quite difficult to develop an ADC formulation. Through a large number of experiments, we have determined the Her2 ADC formulation combination with excellent performance in all aspects. The anti-Her2 monoclonal antibody-MMAE conjugate can dissolve well before and after lyophilization, and both the insoluble microparticles and the visible precipitates meet the standards for human injection; at the same time, the conjugate can be kept stable for a long time during lyophilization and storage, especially, it still maintains good stability after long-term storage at a high temperature of 25 °C or 37 °C. Furthermore, the ADC formulation is not easily to polymerize or be degraded after reconstitution, and maintains good biological activity.

## Claims

1. An aqueous liquid pharmaceutical formulation of an antibody-drug conjugate, wherein
the formulation comprises the antibody-drug conjugate, a non-reducing sugar, an amino acid, and a solubilizer, the non-reducing sugar is selected from mannitol, sucrose, and a combination thereof, the amino acid is selected from histidine, histidine hydrochloride, and a combination thereof, and the solubilizer is polysorbate 80;
wherein the antibody-drug conjugate is an anti-HER2 monoclonal antibody and the drug is MMAE, and wherein the antibody of the antibody-drug conjugate comprises a heavy chain and a light chain, the heavy chain comprises CDRs 1-3 having amino acid sequences as shown in SEQ ID NO: 1, 2 and 3, respectively, and wherein the light chain comprises CDRs 1-3 having amino acid sequences as shown in SEQ ID NO: 4, 5 and 6, respectively.

2. The aqueous liquid pharmaceutical formulation of claim 1, wherein the anti-HER2 monoclonal antibody is connected to MMAE via a linker.

3. The aqueous liquid pharmaceutical formulation according to claim 1 or 2, wherein the concentration of the mannitol is 100-300 mmol/L, preferably 190-300 mmol/L, more preferably 200-260 mmol/L, and most preferably 240-260 mmol/L; the concentration of the sucrose is 0-100 mmol/L, preferably 40-100 mmol/L, more preferably 60-100 mmol/L, and most preferably 40-60 mmol/L.

4. The aqueous liquid pharmaceutical formulation according to claim 1-3, wherein the amino acid is histidine hydrochloride at a concentration of 0-100 mmol/L, preferably 5-50 mmol/L, more preferably 5-20 mmol/L, and most preferably 10 mmol/L.

5. The aqueous liquid pharmaceutical formulation according to any one of claims 1-4, wherein the mass percentage of polysorbate 80 is 0.02% (w/v).

6. The aqueous liquid pharmaceutical formulation according to claim 1, wherein the monoclonal antibody is a chimeric antibody or a humanized antibody.

7. The aqueous liquid pharmaceutical formulation according to any one of claims 1-6, wherein the formulation has a pH of 4.5-7, preferably 5.6-6.8, more preferably 5.6-6.5, 5.6-6.4, 5.6-6.3, 6.1-6.4, or 6.1-6.3.

8. A lyophilized pharmaceutical formulation of an antibody-drug conjugate, wherein the lyophilized pharmaceutical formulation is obtained by lyophilization of the aqueous liquid pharmaceutical formulation according to any one of claims 1-7.

9. The pharmaceutical formulation according to any one of claims 1-8 for use in treating a disease caused by abnormal expression of Her2, preferably cancer, further preferably Her2 positive cancer, and most preferably breast cancer, ovarian cancer, gastric cancer, urothelial cancer, gastroesophageal cancer, oesophageal cancer, endometrial cancer, lung cancer, or bladder cancer.

10. A method for preparing a pharmaceutical formulation of an antibody-drug conjugate, comprising:
(1) preparing the formulation of any one of claims 1-8; and
(2) evaluating the stability of the antibody-drug conjugate in the formulation.

## Patentansprüche

1. Wässrige flüssige pharmazeutische Formulierung eines Antikörper-Arzneimittel-Konjugats, wobei die Formulierung das Antikörper-Arzneimittel-Konjugat, einen nicht-reduzierenden Zucker, eine Aminosäure und einen Lösungsvermittler umfasst, wobei der nicht-reduzierende Zucker aus Mannit, Saccharose und einer Kombination davon ausgewählt ist, die Aminosäure aus Histidin, Histidinhydrochlorid und einer Kombination davon ausgewählt ist, und der Lösungsvermittler Polysorbat 80 ist;
wobei das Antikörper-Arzneimittel-Konjugat ein monoklonaler Anti-HER2-Antikörper ist und das Arzneimittel MMAE ist, und wobei der Antikörper des Antikörper-Arzneimittel-Konjugats eine schwere Kette und eine leichte Kette umfasst, wobei die schwere Kette CDRs 1-3 mit Aminosäuresequenzen umfasst, wie in SEQ ID NO: 1, 2 beziehungsweise 3 gezeigt, und wobei die leichte Kette CDRs 1-3 mit Aminosäuresequenzen umfasst, wie in SEQ ID NO: 4, 5 beziehungsweise 6 gezeigt.

2. Wässrige flüssige pharmazeutische Formulierung nach Anspruch 1, wobei der monoklonale Anti-HER2-Antikörper über einen Linker mit MMAE verbunden ist.

3. Wässrige flüssige pharmazeutische Formulierung nach Anspruch 1 oder 2, wobei die Konzentration des Mannits 100-300 mmol/l, vorzugsweise 190-300 mmol/l, stärker bevorzugt 200-260 mmol/l und am stärksten bevorzugt 240-260 mmol/l ist; die Konzentration der Saccharose 0-100 mmol/l, vorzugsweise 40-100 mmol/l, stärker bevorzugt 60-100 mmol/l und am stärksten bevorzugt 40-60 mmol/l ist.

4. Wässrige flüssige pharmazeutische Formulierung nach Anspruch 1-3, wobei die Aminosäure Histidinhydrochlorid in einer Konzentration von 0-100 mmol/l, vorzugsweise 5-50 mmol/l, stärker bevorzugt 5-20 mmol/l, und am stärksten bevorzugt 10 mmol/l ist.

5. Wässrige flüssige pharmazeutische Formulierung nach einem der Ansprüche 1-4, wobei der Massenprozentsatz von Polysorbat 80 0,02 % (Gew./Vol.) beträgt.

6. Wässrige flüssige pharmazeutische Formulierung nach Anspruch 1, wobei der monoklonale Antikörper ein chimärer Antikörper oder ein humanisierter Antikörper ist.

7. Wässrige flüssige pharmazeutische Formulierung nach einem der Ansprüche 1-6, wobei die Formulierung einen pH-Wert von 4,5-7, vorzugsweise von 5,6-6,8, stärker bevorzugt von 5,6-6,5, 5,6-6,4, 5,6-6,3, 6,1-6,4 oder 6.,1-6,3 aufweist.

8. Lyophilisierte pharmazeutische Formulierung eines Antikörper-Arzneimittel-Konjugats, wobei die lyophilisierte pharmazeutische Formulierung durch Lyophilisierung der wässrigen flüssigen pharmazeutischen Formulierung nach einem der Ansprüche 1-7 erhalten wird.

9. Pharmazeutische Formulierung nach einem der Ansprüche 1-8 für die Verwendung bei der Behandlung einer Krankheit, die durch abnorme Expression von Her2 verursacht wird, vorzugsweise Krebs, ferner vorzugsweise Her2-positiver Krebs, und am stärksten bevorzugt Brustkrebs, Eierstockkrebs, Magenkrebs, Urothelkarzinom, gastroösophagealer Krebs, Speiseröhrenkrebs, Endometriumkrebs, Lungenkrebs oder Harnblasenkrebs.

10. Verfahren zum Herstellen einer pharmazeutischen Formulierung eines Antikörper-Arzneimittel-Konjugats, umfassend:
(1) Herstellen der Formulierung nach einem der Ansprüche 1-8; und
(2) Bewertung der Stabilität des Antikörper-Arzneimittel-Konjugats in der Formulierung.

## Revendications

1. Préparation pharmaceutique liquide aqueuse d'un conjugué anticorps-médicament, dans laquelle la préparation comprend le conjugué anticorps-médicament, un sucre non réducteur, un acide aminé, et un solubilisant, le sucre non réducteur est sélectionné parmi le mannitol, le saccharose, et une combinaison de ceux-ci, l'acide aminé est sélectionné parmi l'histidine, le chlorhydrate d'histidine de, et une combinaison de ceux-ci, et le solubilisant est le polysorbate 80 ;
dans laquelle le conjugué anticorps-médicament est un anticorps monoclonal anti-HER2 et le médicament est MMAE, et dans laquelle l'anticorps du conjugué anticorps-médicament comprend une chaîne lourde et une chaîne légère, la chaîne lourde comprend des CDRs 1 à 3 ayant des séquences d'acides aminés telles que présentées dans SEQ ID n° : 1, 2, et 3, respectivement, et dans laquelle la chaîne légère comprend des CDRs 1 à 3 ayant des séquences d'acides aminés telles que présentées dans SEQ ID n° : 4, 5, et 6, respectivement.

2. Préparation pharmaceutique liquide aqueuse de la revendication 1, dans laquelle l'anticorps monoclonal anti-HER2 est relié à MMAE par l'intermédiaire d'un lieur.

3. Préparation pharmaceutique liquide aqueuse selon la revendication 1 ou 2, dans laquelle la concentration du mannitol est de 100 à 300 mmol/L, de préférence de 190 à 300 mmol/L, de façon davantage préférée de 200 à 260 mmol/L, et de façon préférée entre toutes de 240 à 260 mmol/L ; la concentration du saccharose est de 0 à 100 mmol/L, de préférence de 40 à 100 mmol/L, de façon davantage préférée de 60 à 100 mmol/L, et de façon préférée entre toutes de 40 à 60 mmol/L.

4. Préparation pharmaceutique liquide aqueuse selon la revendication 1 à 3, dans laquelle l'acide aminé est le chlorhydrate d'histidine à une concentration de 0 à 100 mmol/L, de préférence de 5 à 50 mmol/L, de façon davantage préférée de 5 à 20 mmol/L, et de façon préférée entre toutes de 10 mmol/L.

5. Préparation pharmaceutique liquide aqueuse selon l'une quelconque des revendications 1 à 4, dans laquelle le pourcentage massique du polysorbate 80 est de 0,02 % (p/v).

6. Préparation pharmaceutique liquide aqueuse selon la revendication 1, dans laquelle l'anticorps monoclonal est un anticorps chimérique ou un anticorps humanisé.

7. Préparation pharmaceutique liquide aqueuse selon l'une quelconque des revendications 1 à 6, dans laquelle la préparation a un pH de 4,5 à 7, de préférence de 5,6 à 6,8, de façon davantage préférée de 5,6 à 6,5, de 5,6 à 6,4, de 5,6 à 6,3, de 6,1 à 6,4, ou de 6,1 à 6,3.

8. Préparation pharmaceutique lyophilisée d'un conjugué anticorps-médicament, dans laquelle la préparation pharmaceutique lyophilisée est obtenue par lyophilisation de la préparation pharmaceutique liquide aqueuse selon l'une quelconque des revendications 1 à 7.

9. Préparation pharmaceutique selon l'une quelconque des revendications 1 à 8 pour utilisation dans le traitement d'une maladie causée par expression anormale de Her2, de préférence le cancer, de façon davantage préférée un cancer Her2-positif, et de façon préférée entre toutes le cancer du sein, le cancer ovarien, le cancer gastrique, le cancer urothélial, le cancer gastro-œsophagien, le cancer œsophagien, le cancer endométrial, le cancer du poumon, ou le cancer de la vessie.

10. Procédé pour préparer une préparation pharmaceutique d'un conjugué anticorps-médicament, comprenant les faits :
(1) de préparer la préparation de l'une quelconque des revendications 1 à 8 ; et
(2) d'évaluer la stabilité du conjugué anticorps-médicament dans la préparation.
